(19) Europäisches Patentamt
European Patent Office
Office européen des brevets

(11) EP 4 303 257 A1

(12) EUROPEAN PATENT APPLICATION

(43) Date of publication:
**10.01.2024 Bulletin 2024/02**

(21) Application number: **23184192.5**

(22) Date of filing: **07.07.2023**

(51) International Patent Classification (IPC):
***C08K 5/00*** *(2006.01)* ***C08K 5/18*** *(2006.01)*
***C08L 67/00*** *(2006.01)* ***C08L 67/02*** *(2006.01)*
***C08L 67/04*** *(2006.01)*

(52) Cooperative Patent Classification (CPC):
(C-Sets available)
**C08L 67/02; C08L 67/04;** C08K 2201/018;
C08L 2201/06 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.07.2022 TW 111125824**

(71) Applicant: **Largan Medical Co., Ltd.**
**Taichung City 408 (TW)**

(72) Inventors:
- **CHEN, Wei-Yuan**
  **408 Taichung City (TW)**
- **CHEN, Po-Tsun**
  **408 Taichung City (TW)**
- **LU, Tzu-Rong**
  **408 Taichung City (TW)**
- **CHEN, Rih-Sian**
  **408 Taichung City (TW)**
- **HONG, Yu Jie**
  **408 Taichung City (TW)**
- **TENG, Chun-Hung**
  **408 Taichung City (TW)**

(74) Representative: **Lang, Christian**
**LangPatent Anwaltskanzlei IP Law Firm**
**Ingolstädter Straße 5**
**80807 München (DE)**

# (54) PLASTICIZER, PLASTIC COMPOSITION, AND PLASTIC PRODUCT

(57) A plasticizer, a plastic composition and a plastic product are provided. The plasticizer composition and the plastic product include the plasticizer, and the plasticizer has a biodegradability. The plasticizer has a structure represented by Formula (I), in which each of the symbols thereof is as defined in the specification, and the plasticizer has a central structure.

EP 4 303 257 A1

(52) Cooperative Patent Classification (CPC): (Cont.)

C-Sets
**C08K 5/0016, C08L 67/02;**
**C08K 5/0016, C08L 67/04;**
**C08K 5/18, C08L 67/02;**
**C08K 5/18, C08L 67/04;**
**C08L 67/02, C08K 5/18, C08L 67/02;**
**C08L 67/04, C08K 5/18, C08L 23/06;**
**C08L 67/04, C08K 5/18, C08L 23/12;**
**C08L 67/04, C08K 5/18, C08L 27/06;**
**C08L 67/04, C08K 5/18, C08L 67/02**

## Description

## BACKGROUND

### Technical Field

The present disclosure relates to a plasticizer, a plastic composition and a plastic product. More particularly, the present disclosure relates to a plasticizer, a plastic composition and a plastic product that have a biodegradability.

### Description of Related Art

The plasticizer is a material used to increase the plasticity of the product, and is often used in the plasticization of the plastic product. The commonly used traditional plasticizers are mostly phthalate compounds with structures similar to estrogen, such as DEHP, DBP, DINP, BBP and DIDP, etc. The above plasticizers are non-biodegradable and can cause the abnormal developments of adolescent children easily, and even increase the incidence of the breast cancer and the ovarian cancer. Since the plasticizers are combined with other substances by the non-chemical bonding method in the plastic products, which are easily released from the plastic products. Therefore, the plasticizers will be absorbed by the human body when the plastic products contact with food or skin.

In the recent years, the environmental protection awareness has risen, and if the traditional plastic products are not properly treated, they will damage the environment and the ecology. Thus, more and more biodegradable products are widely promoted, but the biodegradation rate thereof is often not high. Most plasticizers and plastics still cannot be decomposed, and the users will misunderstand that these products will not affect the environment and the ecology by any disposal due to these products are biodegradable, resulting in these products not being properly treated and further damaging the environment and the ecology. Nowadays, the plastic products have become an indispensable part of human life, so that it is very important to find the plasticizers that are not easily released from the plastic products or absorbed by the human body, and to develop the biodegradable plastic products.

## SUMMARY

According to one aspect of the present disclosure, a plasticizer has a biodegradability, and has a structure represented by Formula (I):

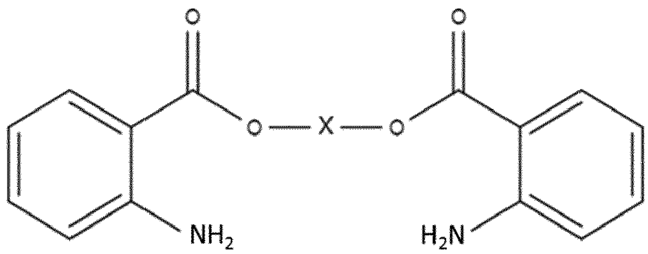

Formula (I),

wherein X is a central structure.

According to the plasticizer of the foregoing aspect, when a number of carbon atoms in X is XC, the following condition can be satisfied: $1 \leq XC \leq 8$.

According to the plasticizer of the foregoing aspect, X can be a straight-chain structure.

According to the plasticizer of the foregoing aspect, when a molecular weight of the plasticizer is Mw, the following condition can be satisfied: 300 g/mole ≤ Mw.

According to the plasticizer of the foregoing aspect, when a pyrolysis temperature of the plasticizer is Pt, the following condition can be satisfied: 1 00°C ≤ Pt.

According to the plasticizer of the foregoing aspect, when a biodegradation rate on a 63rd day is Dr63, the following condition of the biodegradation rate on the 63rd day of the plasticizer can be satisfied: 92% ≤ Dr63.

According to another aspect of the present disclosure, a plastic composition includes at least one plastic and the plasticizer according to the foregoing aspect.

According to the plastic composition of the foregoing aspect, a weight percentage of the at least one plastic in

the plastic composition can be 85% to 99.99%, and a weight percentage of the plasticizer in the plastic composition can be 0.01% to 15%.

According to the plastic composition of the foregoing aspect, the plastic can be a biodegradable plastic.

According to the plastic composition of the foregoing aspect, the plastic composition can further include at least one non-biodegradable plastic, wherein a weight percentage of the at least one non-biodegradable plastic in the plastic composition can be less than a weight percentage of the biodegradable plastic in the plastic composition.

According to the plastic composition of the foregoing aspect, when a biodegradation rate on a 14th day is Dr14, the following condition of the biodegradation rate on the 14th day of the plastic composition can be satisfied: 1% ≤ Dr14.

According to the plastic composition of the foregoing aspect, when a biodegradation rate on a 21st day is Dr21, the following condition of the biodegradation rate on the 21st day of the plastic composition can be satisfied: 5% ≤ Dr21.

According to the plastic composition of the foregoing aspect, when a biodegradation rate on a 28th day is Dr28, the following condition of the biodegradation rate on the 28th day of the plastic composition can be satisfied: 10% ≤ Dr28.

According to the plastic composition of the foregoing aspect, when a biodegradation rate on a 42nd day is Dr42, the following condition of the biodegradation rate on the 42nd day of the plastic composition can be satisfied: 20% ≤ Dr42.

According to further another aspect of the present disclosure, a plastic product includes the plastic composition according to the foregoing aspect.

According to still another aspect of the present disclosure, a plastic composition includes at least one plastic and at least one plasticizer. The plasticizer includes an aminobenzoyl ester compound, and the aminobenzoyl ester compound has a biodegradability. A weight percentage of the plastic in the plastic composition is 85% to 99.99%, and a weight percentage of the plasticizer in the plastic composition is 0.01% to 15%.

According to the plastic composition of the foregoing aspect, the plasticizer can have a structure represented by Formula (I):

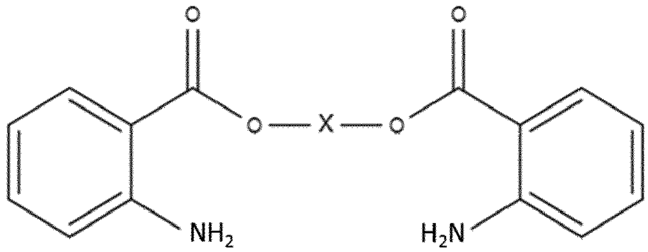

Formula (I),

wherein X is a central structure, and when a number of carbon atoms in X is XC, the following condition can be satisfied: 1 ≤ XC ≤ 8.

According to the plastic composition of the foregoing aspect, when a biodegradation rate on a 14th day is Dr14, the following condition of the biodegradation rate on the 14th day of the plastic composition can be satisfied: 1% ≤ Dr14.

According to the plastic composition of the foregoing aspect, when a biodegradation rate on a 42nd day is Dr42, the following condition of the biodegradation rate on the 42nd day of the plastic composition can be satisfied: 20% ≤ Dr42.

According to yet another aspect of the present disclosure, a plastic product includes the plastic composition according to the foregoing aspect.

## DETAILED DESCRIPTION

The present disclosure provides a plasticizer, which has a biodegradability, and has a structure represented by Formula (I):

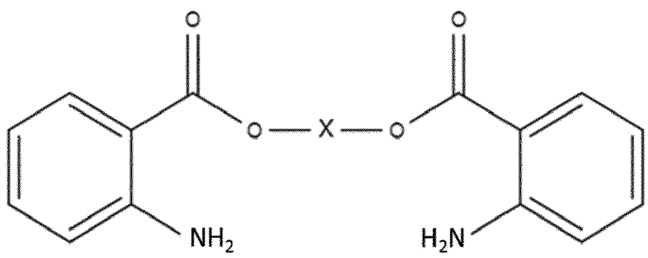

Formula (I),

wherein X is a central structure. Therefore, the damage to the environment and the ecology can be reduced by the characteristics of biodegradable. Further, the plasticizer can have the excellent safety to prevent it from affecting the normal physiological functions of the human body by being designed without a phthalate structure, which is different from the traditional non-biodegradable plasticizer. Furthermore, the methyl anthranilate is a natural compound contained in the fruits, which is edible, so that the plasticizer can have the safer biochemical property by designing the plasticizer structure that is similar to methyl anthranilate.

According to the plasticizer of the present disclosure, in Formula (I), when a number of carbon atoms in X is XC, the following condition can be satisfied: $1 \leq XC \leq 8$. Therefore, the plasticizer has an excellent biodegradation rate that is helped by satisfying the number of carbon atoms in the central structure. Furthermore, the following condition can be satisfied: $2 \leq XC \leq 7$. Furthermore, the following condition can be satisfied: $2 \leq XC \leq 6$. Furthermore, the following condition can be satisfied: $3 \leq XC \leq 5$.

According to the plasticizer of the present disclosure, in Formula (I), X can be a straight-chain structure. Therefore, due to the straight-chain molecule has a higher boiling point than the branched-chain molecule, the plasticizer can be prevented from being adsorbed by the human body after being vaporized by the high temperature.

According to the plasticizer of the present disclosure, when a molecular weight of the plasticizer is Mw, the following condition can be satisfied: 300 g/mole ≤ Mw. Therefore, the plasticizer is not easily absorbed by the human body and can have the excellent safety by designing the plasticizer with the specific molecular weight. Furthermore, the following condition can be satisfied: 305 g/mole ≤ Mw ≤ 500 g/mole. Furthermore, the following condition can be satisfied: 310g/mole ≤ Mw ≤ 450 g/mole. Furthermore, the following condition can be satisfied: 315 g/mole ≤ Mw ≤ 400 g/mole. Furthermore, the following condition can be satisfied: 320 g/mole ≤ Mw ≤ 350 g/mole. Furthermore, the following condition can be satisfied: 325 g/mole ≤ Mw ≤ 330 g/mole.

According to the plasticizer of the present disclosure, when a pyrolysis temperature of the plasticizer is Pt, the following condition can be satisfied: 100°C ≤ Pt. Therefore, the high pyrolysis temperature of the plasticizer can help to stabilize the structure of the plasticizer at the high temperature. Furthermore, the following condition can be satisfied: 150°C ≤ Pt. Furthermore, the following condition can be satisfied: 200°C ≤ Pt. Furthermore, the following condition can be satisfied: 250°C ≤ Pt. Furthermore, the following condition can be satisfied: 300°C ≤ Pt ≤ 500°C.

According to the plasticizer of the present disclosure, when a biodegradation rate on a 63rd day is Dr63, the following condition of the biodegradation rate on the 63rd day of the plasticizer can be satisfied: 92% ≤ Dr63. Therefore, the biodegradation rate of the plasticizer can be improved by satisfying the biodegradation rate on the 63rd day of the plasticizer. Furthermore, the following condition can be satisfied: 93% ≤ Dr63. Furthermore, the following condition can be satisfied: 94% ≤ Dr63. Furthermore, the following condition can be satisfied: 94.5% ≤ Dr63 ≤ 100%.

The present disclosure provides a plastic composition, which includes at least one plastic and the abovementioned plasticizer. Therefore, the damage to the environment and the ecology can be reduced by the plasticizer with biodegradable characteristics.

The present disclosure provides a plastic composition, which includes at least one plastic and at least one plasticizer. The plasticizer includes an aminobenzoyl ester compound, wherein the aminobenzoyl ester compound has a biodegradability. Therefore, the damage to the environment and the ecology can be reduced by the plasticizer with biodegradable characteristics.

According to the plastic composition of the present disclosure, a weight percentage of the plastic in the plastic composition is 85% to 99.99%, and a weight percentage of the plasticizer in the plastic composition is 0.01% to 15%. Therefore, the plastic composition has the good plasticizing effect by a specific mixing ratio. Furthermore, the weight percentage of the plastic in the plastic composition can be 87.5% to 99.99%, and the weight percentage of the plasticizer in the plastic composition can be 0.01% to 12.5%. Furthermore, the weight percentage of the plastic in the plastic composition can be 90% to 99.99%, and the weight percentage of the plasticizer in the plastic composition can be 0.01% to 10%. Furthermore, the weight percentage of the plastic in the plastic composition can be 92.5% to 99.99%, and the

weight percentage of the plasticizer in the plastic composition can be 0.01% to 7.5%. Furthermore, the weight percentage of the plastic in the plastic composition can be 94% to 99.99%, and the weight percentage of the plasticizer in the plastic composition can be 0.01% to 6%.

According to the plastic composition of the present disclosure, the plastic can be a biodegradable plastic. Therefore, the damage to the environment and the ecology can be reduced by the characteristics of biodegradable plastic and plasticizer.

According to the plastic composition of the present disclosure, the plastic composition can further include at least one non-biodegradable plastic. A weight percentage of the non-biodegradable plastic in the plastic composition is less than a weight percentage of the biodegradable plastic in the plastic composition. Therefore, the plastic composition including the non-biodegradable plastic can help to shape the plastic composition and improve the stability of the structure. Further, due to the weight percentage of the non-biodegradable plastic in the plastic composition is less than the weight percentage of the biodegradable plastic in the plastic composition, the plastic composition can be ensured to have a good biodegradation rate.

According to the plastic composition of the present disclosure, when a biodegradation rate on a 14th day is Dr14, the following condition of the biodegradation rate on the 14th day of the plastic composition can be satisfied: 1% ≤ Dr14. Therefore, an initial biodegradation rate of the plastic composition can be improved by satisfying the biodegradation rate on the 14th day of the plastic composition. Furthermore, the following condition can be satisfied: 1.5% ≤ Dr14. Furthermore, the following condition can be satisfied: 2% ≤ Dr14. Furthermore, the following condition can be satisfied: 2.4% ≤ Dr14 ≤ 100%.

According to the plastic composition of the present disclosure, when a biodegradation rate on a 21st day is Dr21, the following condition of the biodegradation rate on the 21st day of the plastic composition can be satisfied: 5% ≤ Dr21. Therefore, a short-term biodegradation rate of the plastic composition can be improved by satisfying the biodegradation rate on the 21st day of the plastic composition. Furthermore, the following condition can be satisfied: 7.5% ≤ Dr21. Furthermore, the following condition can be satisfied: 10% ≤ Dr21. Furthermore, the following condition can be satisfied: 12.5% ≤ Dr21. Furthermore, the following condition can be satisfied: 14% ≤ Dr21 ≤ 100%.

According to the plastic composition of the present disclosure, when a biodegradation rate on a 28th day is Dr28, the following condition of the biodegradation rate on the 28th day of the plastic composition can be satisfied: 10% ≤ Dr28. Therefore, a short-medium term biodegradation rate of the plastic composition can be improved by satisfying the biodegradation rate on the 28th day of the plastic composition. Furthermore, the following condition can be satisfied: 15% ≤ Dr28. Furthermore, the following condition can be satisfied: 20% ≤ Dr28. Furthermore, the following condition can be satisfied: 25% ≤ Dr28. Furthermore, the following condition can be satisfied: 30% ≤ Dr28. Furthermore, the following condition can be satisfied: 35% ≤ Dr28 ≤ 100%.

According to the plastic composition of the present disclosure, when a biodegradation rate on a 42nd day is Dr42, the following condition of the biodegradation rate on the 42nd day of the plastic composition can be satisfied: 20% ≤ Dr42. Therefore, a mid-term biodegradation rate of the plastic composition can be improved by satisfying the biodegradation rate on the 42nd day of the plastic composition. Furthermore, the following condition can be satisfied: 25% ≤ Dr42. Furthermore, the following condition can be satisfied: 30% ≤ Dr42. Furthermore, the following condition can be satisfied: 35% ≤ Dr42. Furthermore, the following condition can be satisfied: 40% ≤ Dr42. Furthermore, the following condition can be satisfied: 45% ≤ Dr42. Furthermore, the following condition can be satisfied: 50% ≤ Dr42 ≤ 100%.

The plasticizer of the present disclosure, the larger molecular weight means that the molecular weight of the plasticizer is greater than 300 g/mole, which is not limited by the plastic materials, and can plasticize different plastics, so that has more wide applicability and convenience.

The plasticizer of the present disclosure can be an aminobenzoyl ester compound. The aminobenzoyl ester compound refers to a compound having an amide group, an ester group and an aminobenzene at the same time. The aminobenzoyl ester compound can be 4-(2-aminobenzoyl)oxybutyl 2-aminobenzoate.

The plasticizer of the present disclosure, in Formula (I), the structure of X can include at least one of carbon atoms, sulfur atoms, oxygen atoms and nitrogen atoms. The bonding of the structure of X can include at least one of carbon-carbon single bonds, carbon-carbon double bonds, carbon-carbon triple bonds, carbon-oxygen bonds, carbon-nitrogen bonds, disulfide bonds, sulfur-oxygen bonds, sulfur-carbon bonds, sulfur-nitrogen bonds, double nitrogen bonds, nitrogen oxygen bonds and double oxygen bonds. The functional group of the structure of X can include at least one of an alkyl group, an alkenyl group, an alkynyl group, a hydroxyl group, a carbonyl group, an aldehyde group, a carbonate group, a carboxyl group, an ether group, an ester group, an amide group, a primary amine group, a secondary amine group, a tertiary amine group, a ketimine group and an aldimine group. Further, XC can be 4, and then the plasticizer is 4-(2-aminobenzoyl)oxybutyl 2-aminobenzoate. XC can be 1, and then the plasticizer is 4-(2-aminobenzoyl)oxymethyl 2-aminobenzoate. XC can be 8, and then the plasticizer is 4-(2-aminobenzoyl)oxyoctyl 2-aminobenzoate.

The plastic composition of the present disclosure can include the biodegradable plasticizer and the biodegradable plastic, and the non-biodegradable plasticizer and the non-biodegradable plastic are added or replaced as required to achieve the balance of function and biodegradable effect. For example, the plastic composition is composed of the

biodegradable plasticizer and the biodegradable plastic, and the non-biodegradable plastic is added, wherein the weight percentage of the non-biodegradable plastic in the plastic composition is less than the weight percentage of the biodegradable plastic in the plastic composition.

The plastic composition of the present disclosure can further include at least one additive, such as a stabilizer, a lubricant, a static coagulant, a flame retardant, a colorant, a filler, an antistatic agent, a light stabilizer, a foaming agent, a coupling agent and a flexibilizer. The stabilizer can be calcium stearate. The lubricant can be fatty acid. The flame retardant can be antimony trioxide. The colorant can be antimony dioxide. The filler can be a calcium carbonate, a glass fiber, an asbestos fiber, a carbon fiber, a boron fiber, a copper disulfide, a glimmer, an asbestos, a silica, a silicate, a metal oxide, a carbon black, a glass bead or a wood flour. The antistatic agent can be stearamidopropyl dimethyl-β-hydroxyethyl amine nitrate. The light stabilizer can be salicylate, benzophenone, benzotriazole, substituted acryl, substituted acrylonitrile, triazine or organic complex. The foaming agent can be azodicarbonamide. Therefore, the characteristic of the plastic, such as the impact resistance, the weather resistance, the heat resistance, the fluidity, the elasticity, the corrosion resistance, the deformation resistance, the light transmittance, the latent modification, the antistatic property, the photolysis resistance, the specific gravity or the toughness can be changed by modifying the plastic properties.

The biodegradable plastic in the plastic composition of the present disclosure can be polylactic acid (PLA), and also can be starch, poly(butyleneadipate-co-terephthalate) (PBAT), polybutylene succinate (PBS), poly propylene carbonate (PPC), polyhydroxyal-kanoates (PHA), polycaprolactone (PCL), polyvinyl alcohol (PVA), polyglycolide (PGA), polyglycerol sebacate (PGS), polyhydroxybutyrate (PHB) or copolymers or mixtures formed from the above polymer monomers.

The non-biodegradable plastic in the plastic composition of the present disclosure can be polyethylene terephthalateco-1,4-cylclohexylenedimethylene terephthalate) (PETG), polyethylene terephthalate (PET), high-density polyethylene (HDPE), polyvinyl chloride (PVC), low-density polyethylene (LDPE), polypropylene (PP), polystyrene (PS), polycarbonate (PC), polymethylmethacrylate (PMMA), acrylonitrile butadiene styrene (ABS), cyclic olefin copolymer (COC), melamine resin or copolymers or mixtures formed from the above polymer monomers.

The condition of the biodegradation rate in the present disclosure uses ASTM-D5338 as a standard method. The percentage of biodegradation rate is calculated by a unit of weight. The degradation product and the activated sludge (fertilizer) are mixed with each other in a weight ratio of 1:6, and the water content in the degradation tank is maintained at about 50% by perlite. The degradation tank is placed at 58 ± 2°C to observe for 180 days. The main components of the compost include livestock manure, soybean flour, bagasse, wood meal, wheat bran and mushroom-raising waste bag, and the components of the perlite are shown in Table 1.

Table 1

| Substance | $SiO_2$ | $Al_2O_3$ | $Fe_2O_3$ | CaO | $K_2O$ | $Na_2O$ | MgO | $H_2O$ |
|---|---|---|---|---|---|---|---|---|
| Content (%) | 68~74 | ~12 | 0.5~3.6 | 0.7~1.0 | 2~3 | 4~5 | 0.3 | 2.3~6.4 |

The fresh 0.1 M KOH aqueous solution is prepared during the testing and monitoring process, and dispensed into a 50 mL of PP tank. Then, the PP tank is placed in the degradation tank, and incubated in a temperate box (58 ± 2°C). The released $CO_2$ during the decomposition of the degradation product is absorbed by the KOH aqueous solution. The replacement frequency of KOH is 6 times/week at the initial stage of degradation (1-30 days), and changed to 2 times/week after one month until satisfied with 180 days. The total test time can also be greater than 180 days. The value of the collected KOH is measured with a pH meter and compared with the control group (0.1 M KOH aqueous solution is placed in the temperate box without degradation reaction), and then convert the hydrogen ion concentration. Then, the weight of $CO_2$ is converted by the hydrogen ion concentration and estimated the biodegradation rate. The conversion formula mentioned above is as follows: hydroxyl concentration change = hydroxyl concentration in the control group - hydroxyl concentration in the experimental group = hydrogen ion concentration. Carbon dioxide weight (unit: mg) = hydrogen ion concentration × 44/2 × 50 mL (44 is the molecular weight of carbon dioxide, 2 is the carbon dioxide reaction equilibrium coefficient, and 50 mL is the volume of aqueous solution). Inorganization (decomposition) percentage = carbon dioxide weight change / theoretical carbon dioxide content for complete decomposition of the composition × 100%.

The biodegradation rate for the nth day in the present disclosure refers to the number of days that the measured object is placed in the degradation tank with ASTM-D5388 as the standard method, wherein the nth day can be the 7th day, the 8th day, the 9th day, the 10th day, the 14th day, the 21st day, the 24th day, the 25th day, the 26th day, the 28th day, the 29th day, the 30th day, the 31st day, the 42nd day, the 52nd day, the 56th day, the 59th day, the 63rd day, the 66th day, the 70th day, the 73rd day, the 77th day, the 80th day, the 84th day, the 87th day, the 91st day, the 94th day, the 98th day, the 101st day, the 150th day, the 154th day, the 157th day, the 175th day, the 178th day, the 182nd day or the 185th day, or can be the biodegradation rate for any day from the 1st day to the 185th day.

Each technical feature in the abovementioned plasticizer and plastic composition can be configured in combination to achieve the corresponding effect.

The present disclosure provides a plastic product, which includes the abovementioned plastic composition. Further, the plastic product of the present disclosure can be a packaging film, a composite paper, a disposable container or a storage utensil, a disposable tableware, a food packaging, a binding tape, a rope, a wrapping film, an agricultural seedling cup and plate, a mushroom space bag, a fertilizer bag, an agricultural tape or belt, an agricultural tiling or a covering material, an agricultural plug tray, a vegetable and a fruit bagging, an agricultural border covering membrane, a surgical needle, a surgical membrane, a bone plate, a drug carrier, an inorganic mixture, a dental filling material, a biological suture anchor, a biological tissue scaffold, a bone nail, a biological tissue filler, a biological suture, a biological experimental petri dish, a biological experimental container, a biological experimental micropipette, a biological experimental centrifuge tube or an experimental glove, etc.

According to the abovementioned embodiments, the specific comparative examples and examples are provided below and described in detail with the drawings.

<1st comparative example>

The plastic composition of the 1st comparative example only includes the plastic, and the plastic contained in the plastic composition is polylactic acid (PLA). Referenced is made to Table 2A, which shows the components and the contents of the plastic composition of the 1st comparative example.

| Table 2A | | |
|---|---|---|
| Plastic | Component | PLA |
| | Weight (g) | 0.5 |
| | Weight percentage (%) | 100 |
| | | |
| Plasticizer | Component | |
| | Weight (g) | 0 |
| | Weight percentage (%) | 0 |
| | | |
| Total weight (g) | | 0.5 |

Referenced is made to Table 2B, which shows the value of the biodegradation rate of the plastic composition of the 1st comparative example at different days.

| Table 2B | |
|---|---|
| Day | Biodegradation rate (%) |
| 0 | 0 |
| 1 | |
| 2 | |
| 3 | 0.00 |
| 4 | |
| 5 | |
| 6 | -0.61 |
| 7 | |
| 8 | |
| 9 | |
| 10 | |

(continued)

| Table 2B | |
|---|---|
| Day | Biodegradation rate (%) |
| 11 | -0.49 |
| 12 | |
| 14 | 2.22 |
| 15 | |
| 16 | |
| 17 | 5.87 |
| 18 | |
| 19 | |
| 21 | 10.97 |
| 22 | |
| 23 | |
| 24 | 15.92 |
| 25 | |
| 26 | |
| 27 | 18.38 |
| 28 | |
| 29 | |
| 30 | |
| 31 | 23.07 |
| 33 | |
| 34 | 28.27 |
| 35 | |
| 38 | 33.71 |
| 39 | |
| 41 | 39.40 |
| 42 | |
| 45 | 45.58 |
| 46 | |
| 48 | 49.41 |
| 49 | |
| 52 | 55.47 |
| 53 | |
| 55 | 60.06 |
| 56 | |
| 59 | 65.67 |
| 60 | |
| 63 | 69.70 |

(continued)

| Table 2B | |
|---|---|
| Day | Biodegradation rate (%) |
| 66 | |
| 67 | 73.06 |
| 70 | |
| 71 | 78.51 |
| 73 | |
| 74 | 79.70 |
| 77 | 79.70 |
| 80 | |
| 81 | 82.56 |
| 84 | |
| 85 | 82.88 |
| 87 | |
| 88 | 83.19 |
| 91 | |
| 94 | |
| 95 | 83.35 |
| 98 | 83.83 |
| 101 | |
| 102 | |
| 103 | 88.54 |
| 105 | 88.77 |
| 106 | |
| 108 | |
| 109 | 90.36 |
| 112 | 91.41 |
| 115 | |
| 116 | 92.46 |
| 119 | 91.07 |
| 122 | |
| 123 | 91.35 |
| 126 | 91.99 |
| 127 | |
| 129 | |
| 130 | 94.13 |
| 131 | |
| 133 | 94.13 |
| 134 | |

(continued)

| Table 2B | |
|---|---|
| Day | Biodegradation rate (%) |
| 136 | |
| 137 | 95.25 |
| 138 | |
| 140 | 96.15 |
| 141 | |
| 143 | |
| 144 | 97.27 |
| 145 | |
| 147 | 97.86 |
| 148 | |
| 150 | |
| 151 | 98.70 |
| 152 | |
| 154 | 99.26 |
| 155 | |
| 157 | |
| 158 | 99.98 |
| 159 | |
| 160 | 100.36 |
| 161 | |
| 162 | |
| 164 | 100.73 |
| 166 | |
| 167 | 100.09 |
| 168 | |
| 169 | |
| 171 | |
| 173 | |
| 174 | 100.68 |
| 175 | |
| 176 | |
| 178 | 100.06 |
| 180 | |
| 182 | |
| 185 | |

<2nd comparative example>

The plastic composition of the 2nd comparative example only includes the plasticizer, and the plasticizer contained in the plastic composition is acetyl tributyl citrate. Referenced is made to Table 3A, which shows the components and the contents of the plastic composition of the 2nd comparative example.

| Table 3A | | |
|---|---|---|
| Plastic | Component | |
| | Weight (g) | 0 |
| | Weight percentage (%) | 0 |
| | | |
| Plasticizer | Component | Acetyl tributyl citrate |
| | Weight (g) | 0.5 |
| | Weight percentage (%) | 100 |
| | | |
| Total weight (g) | | 0.5 |

Referenced is made to Table 3B, which shows the value of the biodegradation rate of the plastic composition of the 2nd comparative example at different days.

| Table 3B | |
|---|---|
| Day | Biodegradation rate (%) |
| 0 | 0 |
| 1 | -0.39 |
| 2 | 2.34 |
| 3 | 7.79 |
| 4 | 9.91 |
| 5 | 11.21 |
| 6 | |
| 7 | 13.32 |
| 8 | 15.86 |
| 9 | 17.21 |
| 10 | 20.95 |
| 11 | 23.59 |
| 12 | 26.13 |
| 14 | 34.84 |
| 15 | 36.90 |
| 16 | 41.84 |
| 17 | 46.53 |
| 18 | 50.72 |
| 19 | 54.99 |
| 21 | 62.09 |
| 22 | 64.49 |

(continued)

| Table 3B | |
|---|---|
| Day | Biodegradation rate (%) |
| 23 | 68.03 |
| 24 | 72.11 |
| 25 | 71.74 |
| 26 | 74.69 |
| 27 | |
| 28 | 81.79 |
| 29 | 83.70 |
| 30 | 84.71 |
| 31 | 85.12 |
| 33 | |
| 34 | |
| 35 | 87.13 |
| 38 | 88.02 |
| 39 | |
| 41 | |
| 42 | 88.81 |
| 45 | 89.32 |
| 46 | |
| 48 | |
| 49 | 89.66 |
| 52 | 90.03 |
| 53 | |
| 55 | |
| 56 | 90.86 |
| 59 | 91.23 |
| 60 | |
| 63 | 91.74 |
| 66 | 92.09 |
| 67 | |
| 70 | 92.46 |
| 71 | |
| 73 | 92.28 |
| 74 | |
| 77 | 92.64 |
| 80 | 93.02 |
| 81 | |
| 84 | 92.83 |

(continued)

| Table 3B | |
|---|---|
| Day | Biodegradation rate (%) |
| 85 | |
| 87 | 92.83 |
| 88 | |
| 91 | 93.00 |
| 94 | 92.62 |
| 95 | |
| 98 | 92.62 |
| 101 | 92.62 |
| 102 | |
| 103 | |
| 105 | |
| 106 | 92.62 |
| 108 | 92.62 |
| 109 | |
| 112 | 92.41 |
| 115 | 91.83 |
| 116 | |
| 119 | 91.83 |
| 122 | 91.83 |
| 123 | |
| 126 | |
| 127 | |
| 129 | 91.27 |
| 130 | |
| 131 | |
| 133 | 90.60 |
| 134 | |
| 136 | 90.11 |
| 137 | |
| 138 | |
| 140 | 89.65 |
| 141 | |
| 143 | 88.68 |
| 144 | |
| 145 | |
| 147 | 88.42 |
| 148 | |

(continued)

| Table 3B | |
|---|---|
| Day | Biodegradation rate (%) |
| 150 | 88.42 |
| 151 | |
| 152 | |
| 154 | 87.87 |
| 155 | |
| 157 | 87.28 |
| 158 | |
| 159 | |
| 160 | |
| 161 | 86.67 |
| 162 | |
| 164 | 86.98 |
| 166 | |
| 167 | |
| 168 | 86.98 |
| 169 | |
| 171 | 86.01 |
| 173 | |
| 174 | |
| 175 | 85.02 |
| 176 | |
| 178 | 84.32 |
| 180 | |
| 182 | 83.70 |
| 185 | 83.35 |

<1st example>

The plastic composition of the 1st example includes the plastic and the plasticizer, and the plastic contained in the plastic composition is polylactic acid. The plasticizer contained in the plastic composition is 4-(2-aminobenzoyl)oxybutyl 2-aminobenzoate, and the details of 4-(2-aminobenzoyl)oxybutyl 2-aminobenzoate are shown in Table 4A.

| Table 4A | |
|---|---|
| Reactant A | 2-aminobenzoic acid |
| Reactant B | 1,4-butanediol |
| Product | 4-(2-aminobenzoyl)oxybutyl 2-aminobenzoate |

(continued)

| Table 4A | |
|---|---|
| Structure | 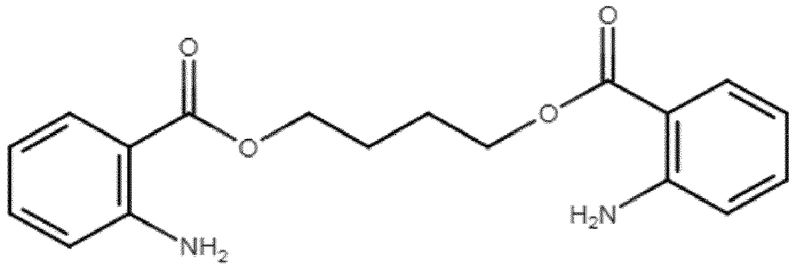 |
| SMILES Notation | NC1=C(C=CC=C1)C(=O)OCCCCOC(=O)C1=C(N)C=CC=C1 |
| Structure schematic | 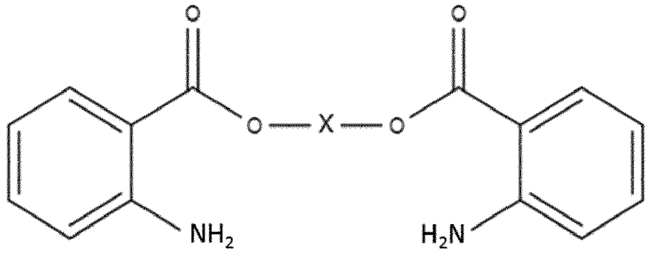 |
| XC | 4 |
| Pt (°C) | 339 |
| Mw (g/mole) | 328 |

Furthermore, referenced is made to Table 4B, which shows the components and the contents of the plastic composition of the 1st example.

| Table 4B | | |
|---|---|---|
| Plastic | Component | PLA |
| | Weight (g) | 0.471 |
| | Weight percentage (%) | 94 |
| | | |
| Plasticizer | Component | 4-(2-aminobenzoyl)oxybutyl 2-aminobenzoate |
| | Weight (g) | 0.029 |
| | Weight percentage (%) | 6 |
| | | |
| Total weight (g) | | 0.5 |

Referenced is made to Table 4C, which shows the value of the biodegradation rate of the plastic composition of the 1st example at different days.

| Table 4C | |
|---|---|
| Day | Biodegradation rate (%) |
| 0 | 0 |
| 1 | -0.16 |
| 2 | 0.04 |

(continued)

| Table 4C | |
|---|---|
| Day | Biodegradation rate (%) |
| 3 | 0.24 |
| 4 | 0.24 |
| 5 | 0.24 |
| 6 | |
| 7 | -0.84 |
| 8 | 0.10 |
| 9 | -0.15 |
| 10 | 0.30 |
| 11 | 0.75 |
| 12 | 1.53 |
| 14 | 2.47 |
| 15 | 3.70 |
| 16 | 5.15 |
| 17 | 6.46 |
| 18 | 7.57 |
| 19 | 8.77 |
| 21 | 14.90 |
| 22 | 17.31 |
| 23 | 21.61 |
| 24 | 26.43 |
| 25 | 27.56 |
| 26 | 28.72 |
| 27 | |
| 28 | 35.21 |
| 29 | 36.59 |
| 30 | 38.56 |
| 31 | 39.25 |
| 33 | |
| 34 | |
| 35 | 44.58 |
| 38 | 49.01 |
| 39 | |
| 41 | |
| 42 | 52.22 |
| 45 | 53.45 |
| 46 | |
| 48 | |

(continued)

| Table 4C | |
|---|---|
| Day | Biodegradation rate (%) |
| 49 | 54.16 |
| 52 | 54.78 |
| 53 | |
| 55 | |
| 56 | 55.22 |
| 59 | 55.70 |
| 60 | |
| 63 | 55.85 |
| 66 | 56.30 |
| 67 | |
| 70 | 56.30 |
| 71 | |
| 73 | 55.82 |
| 74 | |
| 77 | 55.66 |
| 80 | 55.98 |
| 81 | |
| 84 | 55.65 |
| 85 | |
| 87 | 55.50 |
| 88 | |
| 91 | 55.35 |
| 94 | 55.02 |
| 95 | |
| 98 | 54.73 |
| 101 | 54.36 |
| 102 | |
| 103 | |
| 105 | |
| 106 | 54.01 |
| 108 | 53.84 |
| 109 | |
| 112 | 53.48 |
| 115 | 52.80 |
| 116 | |
| 119 | 52.45 |
| 122 | 52.27 |

(continued)

| Table 4C | |
|---|---|
| Day | Biodegradation rate (%) |
| 123 | |
| 126 | |
| 127 | |
| 129 | 51.79 |
| 130 | |
| 131 | |
| 133 | 51.41 |
| 134 | |
| 136 | 50.99 |
| 137 | |
| 138 | |
| 140 | 50.39 |
| 141 | |
| 143 | 49.55 |
| 144 | |
| 145 | |
| 147 | 49.10 |
| 148 | |
| 150 | 48.88 |
| 151 | |
| 152 | |
| 154 | 48.40 |
| 155 | |
| 157 | 48.15 |
| 158 | |
| 159 | |
| 160 | |
| 161 | 47.62 |
| 162 | |
| 164 | 47.62 |
| 166 | |
| 167 | |
| 168 | 47.34 |
| 169 | |
| 171 | 46.50 |
| 173 | |
| 174 | |

(continued)

| Table 4C | |
|---|---|
| Day | Biodegradation rate (%) |
| 175 | 46.22 |
| 176 | |
| 178 | 45.62 |
| 180 | |
| 182 | 44.80 |
| 185 | 44.20 |

<2nd example>

The plastic composition of the 2nd example only includes the plasticizer, and the plasticizer contained in the plastic composition is 4-(2-aminobenzoyl)oxybutyl 2-aminobenzoate, wherein the details of 4-(2-aminobenzoyl)oxybutyl 2-aminobenzoate are shown in Table 4A, and will not be described herein. Referenced is made to Table 5A, which shows the components and the contents of the plastic composition of the 2nd example.

| Table 5A | | |
|---|---|---|
| Plastic | Component | |
| | Weight (g) | 0 |
| | Weight percentage (%) | 0 |
| | | |
| Plasticizer | Component | 4-(2-aminobenzoyl)oxybutyl 2-aminobenzoate |
| | Weight (g) | 0.5 |
| | Weight percentage (%) | 100 |
| | | |
| Total weight (g) | | 0.5 |

Referenced is made to Table 5B, which shows the value of the biodegradation rate of the plastic composition of the 2nd example at different days.

| Table 5B | |
|---|---|
| Day | Biodegradation rate (%) |
| 0 | 0 |
| 1 | 0.62 |
| 2 | 1.62 |
| 3 | 3.15 |
| 4 | |
| 5 | 2.66 |
| 6 | 3.84 |
| 7 | 3.84 |
| 8 | 5.11 |
| 9 | 4.85 |

(continued)

| Table 5B | |
|---|---|
| Day | Biodegradation rate (%) |
| 10 | |
| 11 | 5.44 |
| 12 | 5.44 |
| 14 | 7.86 |
| 15 | 10.11 |
| 16 | |
| 17 | |
| 18 | 15.13 |
| 19 | |
| 21 | 16.50 |
| 22 | |
| 23 | |
| 24 | |
| 25 | 22.63 |
| 26 | |
| 27 | |
| 28 | 31.16 |
| 29 | |
| 30 | |
| 31 | |
| 33 | 40.07 |
| 34 | |
| 35 | 44.68 |
| 38 | |
| 39 | 56.69 |
| 41 | |
| 42 | 67.62 |
| 45 | |
| 46 | 79.63 |
| 48 | |
| 49 | 82.44 |
| 52 | |
| 53 | 86.42 |
| 55 | |
| 56 | 90.28 |
| 59 | |
| 60 | 94.13 |

(continued)

| Table 5B | |
|---|---|
| Day | Biodegradation rate (%) |
| 63 | 94.86 |
| 66 | |
| 67 | 95.59 |
| 70 | 96.10 |
| 71 | |
| 73 | |
| 74 | 96.53 |
| 77 | 96.44 |
| 80 | |
| 81 | 96.53 |
| 84 | 96.33 |
| 85 | |
| 87 | |
| 88 | 95.84 |
| 91 | 95.74 |
| 94 | |
| 95 | 95.34 |
| 98 | 96.22 |
| 101 | |
| 102 | 96.22 |
| 103 | |
| 105 | 96.44 |
| 106 | |
| 108 | |
| 109 | 96.51 |
| 112 | 96.69 |
| 115 | |
| 116 | 97.16 |
| 119 | 97.33 |
| 122 | |
| 123 | 98.20 |
| 126 | |
| 127 | 98.20 |
| 129 | |
| 130 | |
| 131 | 98.58 |
| 133 | |

(continued)

| Table 5B | |
|---|---|
| Day | Biodegradation rate (%) |
| 134 | 97.97 |
| 136 | |
| 137 | |
| 138 | 97.80 |
| 140 | |
| 141 | 97.50 |
| 143 | |
| 144 | |
| 145 | 97.26 |
| 147 | |
| 148 | 97.12 |
| 150 | |
| 151 | |
| 152 | 98.17 |
| 154 | |
| 155 | 98.27 |
| 157 | |
| 158 | |
| 159 | 98.75 |
| 160 | |
| 161 | |
| 162 | 100.35 |
| 164 | |
| 166 | 100.12 |
| 167 | |
| 168 | |
| 169 | 98.56 |
| 171 | |
| 173 | 100.11 |
| 174 | |
| 175 | |
| 176 | 100.28 |
| 178 | |
| 180 | 100.11 |
| 182 | |
| 185 | |

<3rd example>

The plastic composition of the 3rd example includes the plastic and the plasticizer, the plastic contained in the plastic composition is polylactic acid, and the plasticizer contained in the plastic composition is 4-(2-aminobenzoyl)oxybutyl 2-aminobenzoate, wherein the details of 4-(2-aminobenzoyl)oxybutyl 2-aminobenzoate are shown in Table 4A, and will not be described herein. Referenced is made to Table 6, which shows the components and the contents of the plastic composition of the 3rd example.

| Table 6 | | |
|---|---|---|
| Plastic | Component | PLA |
| | Weight (g) | 0.475 |
| | Weight percentage (%) | 95 |
| | | |
| Plasticizer | Component | 4-(2-aminobenzoyl)oxybutyl 2-aminobenzoate |
| | Weight (g) | 0.025 |
| | Weight percentage (%) | 5 |
| | | |
| Total weight (g) | | 0.5 |

<4th example>

The plastic composition of the 4th example includes the plastic and the plasticizer, the plastic contained in the plastic composition is polylactic acid, and the plasticizer contained in the plastic composition is 4-(2-aminobenzoyl)oxybutyl 2-aminobenzoate, wherein the details of 4-(2-aminobenzoyl)oxybutyl 2-aminobenzoate are shown in Table 4A, and will not be described herein. Referenced is made to Table 7, which shows the components and the contents of the plastic composition of the 4th example.

| Table 7 | | |
|---|---|---|
| Plastic | Component | PLA |
| | Weight (g) | 0.48 |
| | Weight percentage (%) | 96 |
| | | |
| Plasticizer | Component | 4-(2-aminobenzoyl)oxybutyl 2-aminobenzoate |
| | Weight (g) | 0.02 |
| | Weight percentage (%) | 4 |
| | | |
| Total weight (g) | | 0.5 |

<5th example>

The plastic composition of the 5th example includes the plastic and the plasticizer, the plastic contained in the plastic composition is polylactic acid, and the plasticizer contained in the plastic composition is 4-(2-aminobenzoyl)oxybutyl 2-aminobenzoate, wherein the details of 4-(2-aminobenzoyl)oxybutyl 2-aminobenzoate are shown in Table 4A, and will not be described herein. Referenced is made to Table 8, which shows the components and the contents of the plastic composition of the 5th example.

| Table 8 | | |
|---|---|---|
| Plastic | Component | PLA |
| | Weight (g) | 0.485 |
| | Weight percentage (%) | 97 |
| | | |
| Plasticizer | Component | 4-(2-am inobenzoyl)oxybutyl 2-aminobenzoate |
| | Weight (g) | 0.015 |
| | Weight percentage (%) | 3 |
| | | |
| Total weight (g) | | 0.5 |

<6th example>

The plastic composition of the 6th example includes the plastic and the plasticizer, the plastic contained in the plastic composition is polylactic acid, and the plasticizer contained in the plastic composition is 4-(2-aminobenzoyl)oxybutyl 2-aminobenzoate, wherein the details of 4-(2-aminobenzoyl)oxybutyl 2-aminobenzoate are shown in Table 4A, and will not be described herein. Referenced is made to Table 9, which shows the components and the contents of the plastic composition of the 6th example.

| Table 9 | | |
|---|---|---|
| Plastic | Component | PLA |
| | Weight (g) | 0.49 |
| | Weight percentage (%) | 98 |
| | | |
| Plasticizer | Component | 4-(2-am inobenzoyl)oxybutyl 2-aminobenzoate |
| | Weight (g) | 0.01 |
| | Weight percentage (%) | 2 |
| | | |
| Total weight (g) | | 0.5 |

<7th example>

The plastic composition of the 7th example includes the plastic and the plasticizer, the plastic contained in the plastic composition is polylactic acid, and the plasticizer contained in the plastic composition is 4-(2-aminobenzoyl)oxybutyl 2-aminobenzoate, wherein the details of 4-(2-aminobenzoyl)oxybutyl 2-aminobenzoate are shown in Table 4A, and will not be described herein. Referenced is made to Table 10, which shows the components and the contents of the plastic composition of the 7th example.

| Table 10 | | |
|---|---|---|
| Plastic | Component | PLA |
| | Weight (g) | 0.495 |
| | Weight percentage (%) | 99 |
| | | |

(continued)

| Table 10 | | |
|---|---|---|
| Plasticizer | Component | 4-(2-am inobenzoyl)oxybutyl 2-aminobenzoate |
| | Weight (g) | 0.005 |
| | Weight percentage (%) | 1 |
| | | |
| Total weight (g) | | 0.5 |

<8th example>

The plastic composition of the 8th example includes the plastic and the plasticizer, the plastic contained in the plastic composition is polyethylene terephthalateco-1,4-cylclohexylenedimethylene terephthalate) (PETG), and the plasticizer contained in the plastic composition is 4-(2-aminobenzoyl)oxybutyl 2-aminobenzoate, wherein the details of 4-(2-aminobenzoyl)oxybutyl 2-aminobenzoate are shown in Table 4A, and will not be described herein. Referenced is made to Table 11, which shows the components and the contents of the plastic composition of the 8th example.

| Table 11 | | |
|---|---|---|
| Plastic | Component | PETG |
| | Weight (g) | 0.475 |
| | Weight percentage (%) | 95 |
| | | |
| Plasticizer | Component | 4-(2-am inobenzoyl)oxybutyl 2-aminobenzoate |
| | Weight (g) | 0.025 |
| | Weight percentage (%) | 5 |
| | | |
| Total weight (g) | | 0.5 |

## Claims

1. A plasticizer, **characterized in** having a biodegradability, and the plasticizer having a structure represented by Formula (I):

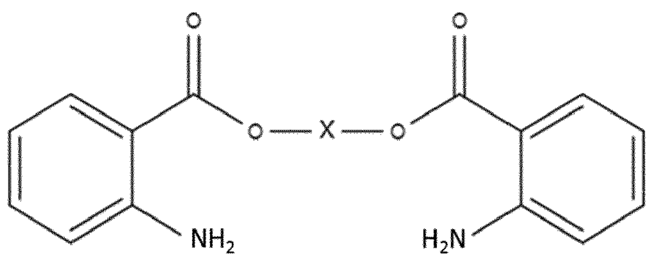

Formula (I),

wherein X is a central structure.

2. The plasticizer of claim 1, wherein a number of carbon atoms in X is XC, and the following condition is satisfied:

$$1 \leq XC \leq 8.$$

3. The plasticizer of claims 1 or 2, wherein X is a straight-chain structure.

4. The plasticizer of any one of claims 1 to 3, wherein a molecular weight of the plasticizer is Mw, and the following condition is satisfied:

$$300\ g/mole \leq Mw.$$

5. The plasticizer of any one of claims 1 to 4, wherein a pyrolysis temperature of the plasticizer is Pt, and the following condition is satisfied:

$$100^{o}C \leq Pt.$$

6. The plasticizer of any one of claims 1 to 5, wherein a biodegradation rate on a 63rd day is Dr63, and the following condition of the biodegradation rate on the 63rd day of the plasticizer is satisfied:

$$92\% \leq Dr63.$$

7. A plastic composition, **characterized in** comprising:

   at least one plastic; and
   the plasticizer of any one of claims 1 to 6.

8. The plastic composition of claim 7, wherein a weight percentage of the at least one plastic in the plastic composition is 85% to 99.99%, and a weight percentage of the plasticizer in the plastic composition is 0.01% to 15%.

9. The plastic composition of claims 7 or 8, wherein the plastic is a biodegradable plastic.

10. The plastic composition of any one of claims 7 to 9, further comprising:
at least one non-biodegradable plastic, wherein a weight percentage of the at least one non-biodegradable plastic in the plastic composition is less than a weight percentage of the biodegradable plastic in the plastic composition.

11. The plastic composition of any one of claims 7 to 10, wherein a biodegradation rate on a 14th day is Dr14, and the following condition of the biodegradation rate on the 14th day of the plastic composition is satisfied:

$$1\% \leq Dr14.$$

12. The plastic composition of any one of claims 7 to 11, wherein a biodegradation rate on a 21st day is Dr21, and the following condition of the biodegradation rate on the 21st day of the plastic composition is satisfied:

$$5\% \leq Dr21.$$

13. The plastic composition of any one of claims 7 to 12, wherein a biodegradation rate on a 28th day is Dr28, and the following condition of the biodegradation rate on the 28th day of the plastic composition is satisfied:

$$10\% \leq Dr28.$$

14. The plastic composition of any one of claims 7 to 13, wherein a biodegradation rate on a 42nd day is Dr42, and the following condition of the biodegradation rate on the 42nd day of the plastic composition is satisfied:

$$20\% \leq Dr42.$$

**15.** A plastic product, **characterized in** comprising:
the plastic composition of any one of claims 7 to 14.

**16.** A plastic composition, **characterized in** comprising:

at least one plastic; and
at least one plasticizer comprising an aminobenzoyl ester compound, wherein the aminobenzoyl ester compound has a biodegradability;
wherein a weight percentage of the at least one plastic in the plastic composition is 85% to 99.99%, and a weight percentage of the at least one plasticizer in the plastic composition is 0.01% to 15%.

**17.** The plastic composition of claim 16, wherein the plasticizer has a structure represented by Formula (I):

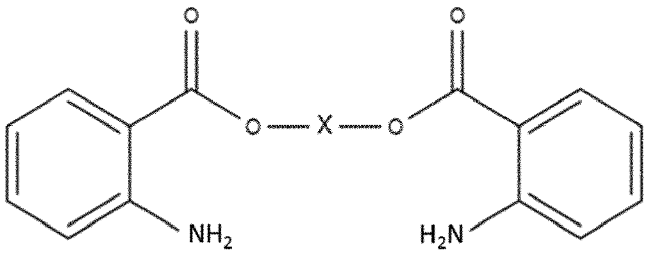

Formula (I),

wherein X is a central structure, and a number of carbon atoms in X is XC, and the following condition is satisfied:

$$1 \leq XC \leq 8.$$

**18.** The plastic composition of claims 16 or 17, wherein a biodegradation rate on a 14th day is Dr14, and the following condition of the biodegradation rate on the 14th day of the plastic composition is satisfied:

$$1\% \leq Dr14.$$

**19.** The plastic composition of any one of claims 16 to 18, wherein a biodegradation rate on a 42nd day is Dr42, and the following condition of the biodegradation rate on the 42nd day of the plastic composition is satisfied:

$$20\% \leq Dr42.$$

**20.** A plastic product, **characterized in** comprising:
the plastic composition of any one of claims 16 to 19.

Europäisches Patentamt
European Patent Office
Office européen des brevets

# EUROPEAN SEARCH REPORT

Application Number
EP 23 18 4192

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 462 060 A2 (CIBA GEIGY AG [CH]) 18 December 1991 (1991-12-18) * abstract; claim 1 * * examples * | 1-15, 17-20 | INV. C08K5/00 C08K5/18 C08L67/00 C08L67/02 C08L67/04 |
| X | US 4 238 351 A (BAYER AG) 9 December 1980 (1980-12-09) * claims 1,2 * | 1-6 | |
| X | WO 2010/053128 A1 (NISSAN CHEMICAL IND LTD [JP]; NAKAHARA SHOICHIRO [JP] ET AL.) 14 May 2010 (2010-05-14) * abstract; compounds 1-14, 1-15 * | 1-6 | |
| X | WO 2018/009737 A1 (SAINT GOBAIN ABRASIVES INC [US]; SAINT GOBAIN ABRASIFS SA [FR] ET AL.) 11 January 2018 (2018-01-11) * abstract; claims 1,9,14 * | 16,18-20 | |
| X | WO 2018/008003 A1 (UNIV MASSACHUSETTS [US]) 11 January 2018 (2018-01-11) * abstract; claim 1; compounds (I),(II),(III) * | 16,18-20 | TECHNICAL FIELDS SEARCHED (IPC) C09J C08K C08L |

The present search report has been drawn up for all claims

| Place of search | Date of completion of the search | Examiner |
|---|---|---|
| Munich | 21 November 2023 | Schütte, Maya |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 23 18 4192

This annex lists the patent family members relating to the patent documents cited in the above-mentioned European search report. The members are as contained in the European Patent Office EDP file on
The European Patent Office is in no way liable for these particulars which are merely given for the purpose of information.

21-11-2023

| Patent document cited in search report | Publication date | Patent family member(s) | Publication date |
|---|---|---|---|
| EP 0462060 A2 | 18-12-1991 | CA 2044365 A1 | 14-12-1991 |
| | | EP 0462060 A2 | 18-12-1991 |
| | | JP H04226122 A | 14-08-1992 |
| US 4238351 A | 09-12-1980 | DE 2516684 A1 | 28-10-1976 |
| | | FR 2307835 A1 | 12-11-1976 |
| | | JP S51126985 A | 05-11-1976 |
| | | US 4238351 A | 09-12-1980 |
| WO 2010053128 A1 | 14-05-2010 | CN 102203662 A | 28-09-2011 |
| | | CN 103739523 A | 23-04-2014 |
| | | CN 104761471 A | 08-07-2015 |
| | | JP 5333454 B2 | 06-11-2013 |
| | | JP 5733340 B2 | 10-06-2015 |
| | | JP 2013241412 A | 05-12-2013 |
| | | JP WO2010053128 A1 | 05-04-2012 |
| | | KR 20110082589 A | 19-07-2011 |
| | | KR 20150138410 A | 09-12-2015 |
| | | TW 201030059 A | 16-08-2010 |
| | | WO 2010053128 A1 | 14-05-2010 |
| WO 2018009737 A1 | 11-01-2018 | CN 109689298 A | 26-04-2019 |
| | | EP 3481589 A1 | 15-05-2019 |
| | | US 2019168358 A1 | 06-06-2019 |
| | | WO 2018009737 A1 | 11-01-2018 |
| WO 2018008003 A1 | 11-01-2018 | CN 109415553 A | 01-03-2019 |
| | | EP 3481899 A1 | 15-05-2019 |
| | | US 2019309159 A1 | 10-10-2019 |
| | | WO 2018008003 A1 | 11-01-2018 |

EPO FORM P0459

For more details about this annex : see Official Journal of the European Patent Office, No. 12/82